(19) Europäisches Patentamt
European Patent Office
Office européen des brevets

(11) **EP 1 799 654 B1**

(12) **EUROPEAN PATENT SPECIFICATION**

(45) Date of publication and mention
of the grant of the patent:
**19.03.2008 Bulletin 2008/12**

(51) Int Cl.:
***C07D 251/54*** (2006.01)

(21) Application number: **05791973.0**

(22) Date of filing: **07.10.2005**

(86) International application number:
**PCT/NL2005/000726**

(87) International publication number:
**WO 2006/041283 (20.04.2006 Gazette 2006/16)**

(54) **COMPOSITION COMPRISING A BINARY MIXTURE OF MELAMINE PARTICLES**

ZUSAMMENSETZUNG MIT EINER BINÄREN MISCHUNG AUS MELAMINPARTIKELN

COMPOSITION COMPRENANT UN MELANGE BINAIRE DE PARTICULES DE MELAMINE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **14.10.2004 EP 04077834**

(43) Date of publication of application:
**27.06.2007 Bulletin 2007/26**

(73) Proprietor: **DSM IP Assets B.V.
6411 TE Heerlen (NL)**

(72) Inventor: **VAN DIJK, Saskia Ingeborg
NL-6002 EJ Weert (NL)**

(56) References cited:
**US-B1- 6 397 494**

**Description**

The invention relates to a composition comprising melamine particles, said composition comprising melamine particles that are obtainable in a crystallising step done on an aqueous phase comprising melamine in solution (melamine A particles),

Such a composition is known, from for example melamine as obtained in the Stamicarbon process for the preparation of melamine as disclosed in section 4.1.3 of the chapter 'Melamine and Guanamines' in Ullmann's Encyclopedia of Industrial Chemistry, Sixth Edition, 2001.

A disadvantage of the known composition is its strong tendency to exhibit caking benaviour. Caking behaviour is the known characteristic of particles to show increased adherence to each other upon storage due to pysical and/or chemical processes. Caking thus leads to difficulties in handling and transport of melamine A particles. An example of such a difficulty is the occurance of problems in removing melamine A particles from big bags or containers due to non-flowing of the particles when the big bags or containers are opened and tilted.

It is the objective of the invention to reduce the said disadvantage.

The objective is achieved in that the composition also comprises melamine particles obtainable in a crystallising step done on a melamine melt or on melamine vapour (melamine D particles).

An advantage of the composition according to the invention is that it shows a reduced caking tendency compared to the known composition, thereby improving the ease of handling and transport of melamine.

The present invention relates to a composition. Within the framework of the present invention, this is understood to mean a mixture of solid particles. The particles as meant herein are solid, and comprise or consist essentially of crystallised melamine - herein referred to as melamine particles. Nevertheless, the melamine particles may comprise, as is known, other compounds such as the compounds that are generated in commercially available processes for the preparation of melamine. Examples of such other compounds are ammeline, ammelide, cyanuric acid, urea, melam, and melem. Typically, the melamine particles consist of melamine of at least 95 wt.% purity, preferably of at least 97 wt. % purity, in particular of melamine of at least 98 or 99 wt.% purity, most preferably of melamine of at least 99.5 wt.% purity.

The dimensions of the particles in the composition according to the invention may vary within wide limits, and are usually co-determined by the method with which the particles were prepared. One customary method for obtaining information on the dimensions of melamine particles in a sample of melamine particles is by determining certain characteristics of the particle size distribution. Such characteristics may be expressed by means of the term 'D$_\alpha$ of β μm'. As is known, this term means that when a particle size distribution measurement is done, the value of 'β' μm is only reached after 'α' wt.% of the particles have been taken into account as undersize fraction. Thus, α wt.% of the particles have a particle size of not more than β μm. Melamine A particles as obtained in the Stamicarbon process typically have a D$_{50}$ between 40 and 140 μm, and a D$_{96}$ between 130 and 300 μm. Melamine D particles - as discussed further below - typically have a D$_{50}$ of between 4-40 μm, and a D$_{96}$ of between 30-130 μm.

The composition according to the invention furthermore comprises melamine particles that are obtainable in a crystallising step done on a melamine melt or on melamine vapour. Such melamine particles are as such known, and are herein referred to as 'melamine D' particles. The crystallising step is preferably done on industrial scale. Melamine D particles are known from for example the Melamine Chemicals process as disclosed in section 4.2.1 of the chapter 'Melamine and Guanamines' in Ullmann's Encyclopedia of Industrial Chemistry, Sixth Edition, 2001, and also from US 4,565,867 or WO 99/46251 A1. In the type of process as disclosed in WO 99/46251 A1, the melamine D particles are obtainable by a process in which the crystallising step comprises the cooling and solidifying of a melamine melt in the form of a spray by means of an evaporating cooling medium, preferably liquid ammonia. These melamine D particles have a multicrystalline character, meaning that essentially all larger particles (>20μm) are composed of a multiplicity of crystals, and that the particles have an average specific surface area lying between 0.7 and 5 m$^2$/g.

Another example of a process by which melamine D particles may be obtained is the type of process as disclosed in WO 95/01345 A1. In the process of WO 95/01345 A1, a melamine melt is prepared from urea; subsequently, the liquid melamine is vaporised. The melamine in vapour form is then directly solidified in a quench step by means of an evaporating cooling liquid (e.g. ammonia).

Yet another example of a process by which melamine D particles may be obtained is the BASF process as described in for example section 4.1.1 of the chapter 'Melamine and Guanamines' in Ullmann's Encyclopedia of Industrial Chemistry, Sixth Edition, 2001. In this type of process, melamine is synthesized catalytically at nearly atmospheric pressures in the gaseous phase, and then filtered to remove catalyst particles and the by-product melem. The crystallising step is then executed, as the filtered melamine-containing gas mixture enters the top of a crystalliser where it is blended countercurrently with a recycled off-gas stream having a temperature of about 140°C. The temperature in the crystalliser is thereby reduced to 190 - 200 °C, and more than 98 % of the melamine crystallizes as fine crystals. The melamine D particles are then recovered from the gas in a cyclone.

Melamine D particles as obtained in the above mentioned processes typically have a D$_{50}$ of 4-40 μm, and a D$_{96}$ of 30-130 μm. It is preferred that no further steps are executed that alter the physical properties of the melamine D particles after they have been prepared. In a preferred embodiment, the melamine D particles in the composition according to the invention consist essentially of multicrystalline particles.

As already indicated above, the composition according to the invention comprises melamine particles obtainable in a crystallising step done on an aqueous phase comprising melamine in solution. Such melamine particles are as such known, and are herein referred to as 'melamine A' particles. The crystallising step is preferably done on industrial scale. Melamine A particles are known from for example the Stamicarbon process as disclosed in section 4.1.3 of the chapter 'Melamine and Guanamines' in Ullmann's Encyclopedia of Industrial Chemistry, Sixth Edition, 2001. In this process, melamine is synthesized catalytically in the gas phase, then obtained as a suspension in aqueous phase followed by complete dissolution in an aqueous phase so that the aqueous phase comprises melamine in solution; in the Stamicarbon process essentially all melamine is dissolved prior to the carrying out of the crystallising step so that the crystallising step as meant in the present invention comprises a recrystallisation operation. In the Stamicarbon process, the crystallising step comprises a solidification step, carried out on the melamine in solution in a vacuum crystallizer; the melamine particles are separated from the aqueous phase by a hydrocyclone and a centrifuge. The melamine particles are then obtained following a drying step in a pneumatic dryer. Melamine particles as obtained in the Stamicarbon process typically have a $D_{50}$ between 40 and 140 $\mu$m, and a $D_{96}$ between 130 and 300 $\mu$m.

Another example of a process comprising a crystallising step done on an aqueous phase comprising melamine in solution as defined in the present invention is the Chemie Linz Process (section 4.1.2 of the chapter 'Melamine and Guanamines' in Ullmann's Encyclopedia of Industrial Chemistry, Sixth Edition, 2001). In the Chemie Linz process, melamine is recovered from the reaction gas in a quench step by quenching with water and with a recycle stream from centrifuges placed further on in the process; this recycle stream is often referred to as mother liquor. As a result of the quench step, a melamine suspension is formed comprising melamine particles and melamine in solution. The suspension is further cooled in a cooling step so as to obtain more melamine particles through crystallisation. After being centrifuged, the melamine particles are obtained by drying. In the Chemie Linz process, the crystallising step as meant in the present invention comprises the quench step and the cooling step.

The melamine A particles are thus obtainable by processes in which a melamine suspension is formed followed by further crystallisation. The melamine A particles are also obtainable by processes that solidify melamine from a solution only. Typically, the aqueous phase (not including the melamine particles) in the crystallizing step contains more than 50 wt.% water, preferably more than 75 wt.% water. It is possible that the melamine A particles, as they have been recovered from the aqueous phase and dried, are subjected to a sieving step in order to remove the largest particles in a typical amount of 0.02 wt.% to 2 wt.%. It is preferred that no further steps are executed that alter the physical properties of the melamine A particles.

The composition according to the invention comprises both melamine A particles and melamine D particles. In the composition, both types of particles should not be in full separation from each other; rather, the composition should comprise a mixture of both types of melamine particles. Thus, in order for the composition according to the invention to come into existence the melamine A particles and the melamine D particles should have been brought together and have undergone - in a mixing step - a degree of mixing with each other. The degree of mixing should be such that the benefits of the present invention, in particular relating to a reduction of caking tendency, are achieved. Mixing of particles is as such known and may be achieved in for example tumble mixers or other suitable equipment known to one skilled in the art, such as a pneumatic transport system; typical mixing times in practice vary greatly and can lie between 3 seconds and 30 minutes.

The amount of the melamine A particles or the melamine D particles in the composition, herein expressed as weight percentage relative to the sum of melamine A and melamine D particles as present in the composition according to the invention, may vary within wide limits. Preferably, however, the amount of melamine A particles lies between 2% and 98% - thus accompanied by between 98% and 2% of melamine D particles. More preferably, the amount of melamine A particles or melamine D particles is at least 5%, 10% or 15%, in particular at least 20% or 25%, more in particular at least 30% or 35%, and most preferably at least 40% or 45 %. In a preferred embodiment of the composition according to the invention, the composition comprises a majority of melamine A particles, whereby the amount of the melamine D particles lies between 5% and 30%, preferably between 10% and 20%. Preferably, the melamine D particles in this embodiment are multicrystalline particles.

A further advantage of the composition according to the invention is that the Tapped Bulk Density (TBD) is higher than would be expected from such a composition. As a result of this, it becomes possible to store more melamine in a storage unit such as a silo, a big bag or a container. Alternatively, smaller storage units can be used that still advantageously contain the same amount of melamine.

The invention also relates to the use of the composition according to the invention in the preparation of melamine-containing aminoplast resins. As is known, the term aminoplast resins refers to an aqueous phase comprising the reaction product of an aldehyde, for example formaldehyde, with an amino compound, i.e. a compound having in unreacted state at least one -$NH_2$ group, preferably at least two -$NH_2$ groups. Examples of aminoplast resins are melamine-formaldehyde resins, urea-formaldehyde resins, and melamine-urea-formaldehyde resins. The composition according to the invention can be used to replace part or all of the known melamine particles used in preparing a melamine-containing aminoplast; the method of administering the composition according to the invention to the aqueous phase can be the same as with the known melamine particles.

Method of determination of caking behaviour

Caking behaviour of a powder composition was evaluated as follows. A round tube having an inner diameter of 36 mm was filled with approximately 250 g of a composition comprising melamine particles. The tube was stored in a climate chamber at 75% relative humidity and underwent a temperature cycle consisting of two steps: 55°C during 2 hours followed by a temperature of 20°C during 2 hours. The cycle was repeated 5 times. After the total storage time the caking tendency of the composition was tested by penetrating the sample with 5 pins (one in the centre and the other four evenly distributed on a circle with a radius of 22 mm concentric to the centre pin) over a length of 45 mm. Each pin had a diameter of 8 mm and a sharp top with a top angle of 10.75°. The work needed to penetrate the sample with this set of pins over the 45 mm length, expressed in millijoules, is an indicator of the caking tendency: the higher the amount of work needed, the higher the caking tendency.

Examples 1 - 3 and Comparative Experiments A and B

Caking behaviour was evaluated according to the method as given above. The melamine A particles as used were prepared by means of the Stamicarbon process; they had a $D_{50}$ of 120 $\mu$m and a $D_{96}$ of 250 $\mu$m. The melamine D particles as used were prepared by means of a process according to US 4,565,867; they had a $D_{50}$ of 6 $\mu$m and a $D_{96}$ of 45 $\mu$m. The results are given in the table below.

| Comparative Experiment / Example | Amount of A particles (wt.%) | Amount of D particles (wt.%) | Work needed for penetration (mJ) |
|---|---|---|---|
| A | 100 | 0 | 265 |
| 1 | 95 | 5 | 151 |
| 2 | 75 | 25 | 137 |
| 3 | 50 | 50 | 148 |
| B | 0 | 100 | 162 |

The results show that a composition comprising 95% melamine A particles and 5% melamine D particles (Example 1) already has a very significant lower caking tendency than a composition consisting of only melamine A particles (Comparative Experiment A); this surprising effect was established even though the melamine D particles as used were significantly smaller than the melamine A particles, a fact which would lead the skilled person to expect an effect in the opposite direction, i.e. an increase in caking behaviour. The results further show that the caking tendency of the compositions according to the invention is also lower than that of pure melamine D particles.

Tapped bulk density measurement method

The measuring cup and other apparatus according to ASTM D1896 (poured bulk density) was used. In addition, the cup was equipped with an extra 'skirt' on top of it so that more material than the content of the cup itself could be poured.
The cup was filled with material, which was poured freely into it. Then, the cup was vibrated; this caused the material to 'settle', leading to a volume reduction. The vibration was stopped when the volume no longer decreased. Then, the 'skirt' was removed, as well as any material still rising above the brim of the cup. Subsequently, the density was calculated from the volume of the cup and the weight of the tapped material inside of it. The result was the tapped bulk density TBD.

Comparative Experiment C

The TBD of a sample of melamine A particles (supplier: DSM) was determined according to the method as described above. The result was 863 kg/m$^3$.

Comparative Experiment D

The TBD of a sample of melamine D particles (supplier: Melamine Chemical Inc) was determined according to the method as described above. The result was 675 kg/m$^3$.

Examples 4 - 6

Compositions comprising the melamine A and D particles in various ratios were prepared by thoroughly mixing the particles in an Erweka SW1/S laboratory high-speed mixer during 20 minutes. The melamine particles were from the same batch of material as was used in the Comparative Experiments A and B. The TBD of each of the compositions was determined by the method as described above.

| Example nr. | Amount of Melamine A particles (%) | Amount of Melamine D particles (%) | Measured TBD (kg/m$^3$) |
|---|---|---|---|
| 4 | 40 | 60 | 780 |
| 5 | 60 | 40 | 864 |
| 6 | 80 | 20 | 934 |

Comparative Experiment E

The TBD of a sample of melamine A particles (supplier: DSM) was determined according to the method as described above. The result was 1019 kg/m$^3$.

Comparative Experiment F

The TBD of a sample of melamine D particles (supplier: Melamine Chemical Inc) was determined according to the method as described above. The result was 811 kg/m$^3$.

Examples 7 - 11

Compositions comprising the melamine A and D particles in various ratios were prepared by thoroughly mixing the particles in an Erweka SW1/S laboratory high-speed mixer during 20 minutes. The melamine particles were from the same batch of material as was used in the Comparative Experiments E and F. The TBD of each of the compositions was determined by the method as described above. Furthermore, the TBD as the skilled person would expect is also given; the expected TBD is calculated from the specific volume calculation as given by Yu, A.B, N.Standish, and A. MecLean, "Porosity Calculation of Binary Mixtures of Nonspherical Particles", J.Amer.Ceram.Soc., 76, 2813(1993). The said calculation uses the following formula (I):

$$\left(\frac{V-V_l X_l}{V_s}\right)^2 + 2G\left(\frac{V-V_l X_l}{V_s}\right)\left(\frac{V-X_l-V_s X_s}{V_l-1}\right) + \left(\frac{V-X_l-V_s X_s}{V_l-1}\right)^2 = 1 \qquad \text{(I)}$$

In formula I, $V$ is the specific volume, $X$ the volume fraction, G an interaction parameter set at 1, subscript $l$ refers to the large component and subscript $s$ refers to the small component. The results are given in the table below.

| Example nr. | Amount of Melamine A particles (%) | Amount of Melamine D particles (%) | Expected TBD (kg/m$^3$) | Measured TBD (kg/m$^3$) |
|---|---|---|---|---|
| 7 | 37.5 | 62.5 | 878 | 938 |
| 8 | 50 | 50 | 903 | 982 |
| 9 | 62.5 | 37.5 | 930 | 985 |
| 10 | 75 | 25 | 958 | 1028 |
| 11 | 87.5 | 12.5 | 987 | 1031 |

Comparative Experiment G

The TBD of a sample of melamine A particles (supplier: DSM) was determined according to the method as described above. The result was 930 kg/m$^3$.

Comparative Experiment H

The TBD of a sample of melamine D particles (supplier: BASF) was determined according to the method as described above. The result was 678 kg/m$^3$.

Examples 12-14

Compositions comprising the melamine A and D particles in various ratios were prepared by thoroughly mixing the particles in an Erweka SW1/S laboratory high-speed mixer during 20 minutes. The melamine particles were from the same batch of material as was used in the Comparative Experiments G and H. The TBD of each of the compositions was determined by the method as described above. The results are given in the table below, as well as the expected TBD of each example, calculated by the Yu method as referred to above.

| Example nr. | Amount of Melamine A particles (%) | Amount of Melamine D particles (%) | Expected TBD (kg/m$^3$) | Measured TBD (kg/m$^3$) |
|---|---|---|---|---|
| 12 | 60 | 40 | 809 | 849 |
| 13 | 80 | 20 | 865 | 952 |
| 14 | 90 | 10 | 897 | 1002 |

As is clear from the examples and comparative experiments, the TBD of the compositions according to the invention is surprisingly higher than the TBD as expected by the skilled person.

**Claims**

1. Composition comprising melamine particles, said composition comprising melamine particles that are obtainable in a crystallising step done on an aqueous phase comprising melamine in solution (melamine A particles), whereby the composition also comprises melamine particles obtainable in a crystallising step done on a melamine melt or on melamine vapour (melamine D particles).

2. Composition according to claim 1, wherein the amount of the melamine D particles lies between 2 wt.% and 30 wt.% of the sum of the amount of melamine A particles and melamine D particles.

3. Composition according to claim 1 or 2, wherein the melamine D particles consist essentially of multicrystalline particles.

4. Process for the preparation of a composition according to any one of claims 1 - 3, wherein melamine A particles and melamine D particles are brought together and mixed.

5. Use of the composition according to any one of claims 1-3 in the preparation of melamine-containing aminoplast resins.

**Patentansprüche**

1. Melaminteilchen enthaltende Zusammensetzung, wobei die Zusammensetzung Melaminteilchen enthält, die in einem an einer Melamin in Lösung enthaltenden wäßrigen Phase durchgeführten Kristallisationsschritt erhältlich sind (Melamin-A-Teilchen), wobei die Zusammensetzung auch Melaminteilchen enthält, die in einem an einer Melaminschmelze oder an Melamindampf durchgeführten Kristallisationsschritt erhältlich sind (Melamin-D-Teilchen).

**2.** Zusammensetzung nach Anspruch 1, in der die Menge der Melamin-D-Teilchen zwischen 2 Ges.-% und 30 Ges.-% der Summe der Menge der Melamin-A-Teilchen und Melamin-D-Teilchen liegt.

**3.** Zusammensetzung nach Anspruch 1 oder 2, in der die Melamin-D-Teilchen im wesentlichen aus multikristallinen Teilchen bestehen.

**4.** Verfahren zur Herstellung einer Zusammensetzung nach einem der Ansprüche 1-3, bei dem man Melamin-A-Teilchen und Melamin-D-Teilchen zusammenbringt und vermischt.

**5.** Verwendung der Zusammensetzung nach einem der Ansprüche 1-3 bei der Herstellung von Melamin enthaltenden Aminoplastharzen.

**Revendications**

**1.** Composition comprenant des particules de mélamine, ladite composition comprenant des particules de mélamine qui peuvent être obtenues dans une étape de cristallisation réalisée sur une phase aqueuse comprenant de la mélamine en solution (particules de mélamine A), la composition comprenant ainsi également des particules de mélamine pouvant être obtenues dans une étape de cristallisation réalisée sur une masse fondue de mélamine ou sur de la vapeur de mélamine (particules de mélamine D).

**2.** Composition selon la revendication 1, dans laquelle la quantité des particules de mélamine D est comprise entre 2 % en poids et 30 % en poids de la somme de la quantité des particules de mélamine A et des particules de mélamine D.

**3.** Composition selon la revendication 1 ou 2, dans laquelle les particules de mélamine D sont constituées essentiellement de particules multi-cristallines.

**4.** Procédé de préparation d'une composition selon l'une quelconque des revendications 1 à 3, dans lequel les particules de mélamine A et les particules de mélamine D sont mises en contact et mélangées.

**5.** Utilisation de la composition selon l'une quelconque des revendications 1 à 3 pour la préparation de résines aminoplastes contenant de la mélamine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4565867 A
- WO 9946251 A1
- WO 9501345 A1

**Non-patent literature cited in the description**

- Melamine and Guanamines. Ullmann's Encyclopedia of Industrial Chemistry. 2001
- **YU, A.B ; N.STANDISH ; A. MECLEAN.** Porosity Calculation of Binary Mixtures of Nonspherical Particles. *J.Amer.Ceram.Soc.,* 1993, vol. 76, 2813